# EUROPÄISCHE PATENTANMELDUNG

(11) **EP 1 681 031 A1**
(43) Veröffentlichungstag der Anmeldung: **19.07.2006**
(21) Anmeldenummer: 06000447.0
(22) Anmeldetag: 11.01.2006
(51) Int. Cl.: A61B 19/00

(54) **Chirurgisches System und Steuergerät für ein chirurgisches System**

(30) Priorität: 18.01.2005 DE 102005003784
(71) Anmelder: Aesculap AG & Co. KG, 78532 Tuttlingen (DE)
(72) Erfinder: Prädel, Christian, 78532 Tuttlingen (DE)
(74) Vertreter: Hoeger, Stellrecht & Partner Patentanwälte

(57) **Zusammenfassung**

Um ein chirurgisches Steuergerät für ein mindestens ein chirurgisches Gerät umfassendes chirurgisches System, wobei das chirurgische System und/oder das mindestens eine chirurgische Gerät mit dem Steuergerät drahtlos steuer- und/oder regelbar ist beziehungsweise sind, so zu verbessern, daß bei einer Erweiterung des Systems das Steuergerät auf einfache Weise entsprechend angepaßt werden kann, wird vorgeschlagen, daß das Steuergerät ein Taschencomputer ist. Ferner wird ein verbessertes chirurgisches System vorgeschlagen.

## Beschreibung

Die vorliegende Erfindung betrifft ein chirurgisches Steuergerät für ein mindestens ein chirurgisches Gerät umfassendes chirurgisches System, wobei das chirurgische System und/oder das mindestens eine chirurgische Gerät mit dem Steuergerät drahtlos steuer- und/oder regelbar ist beziehungsweise sind.

Ferner betrifft die vorliegende Erfindung ein chirurgisches System umfassend mindestens ein chirurgisches Gerät und ein chirurgisches Steuergerät zum drahtlosen Steuern und/oder Regeln des chirurgischen Systems.

Zur Bedienung von chirurgischen Geräten ist es bekannt, diese zu einem Gesamtsystem zu verbinden. Dies ermöglicht einen individuellen, vorzugsweise modularen Aufbau des Systems. Dabei werden Steuergeräte eingesetzt, mit denen das Gesamtsystem oder auch einzelne Geräte bedienbar sind, das heißt gesteuert und/oder geregelt werden können. Die Kommunikation zwischen dem Steuergerät und dem System beziehungsweise den Geräten des Systems erfolgt über Kabelverbindungen oder auch drahtlos. Allen bekannten Systemen ist gemein, daß ein speziell für das System beziehungsweise das mindestens eine Gerät ausgebildetes Steuergerät vorgesehen sein muß. Die spezielle Ausbildung des Steuergeräts führt dann insbesondere bei einer Erweiterung des Systems zu dem Problem, daß mit dem Steuergerät in der Regel nicht alle Funktionen des zusätzlichen Geräts steuer- und/oder regelbar sind und daher zur optimalen Nutzung des Systems ein erweitertes Steuergerät benötigt wird. Dabei entstehen häufig sehr hohe Kosten, insbesondere dann, wenn am Steuergerät neue Bedienelemente vorgesehen werden müssen, es also unter Umständen völlig neu konstruiert werden muß.

Es ist daher Aufgabe der vorliegenden Erfindung, ein chirurgisches Steuergerät und ein chirurgisches System der eingangs beschriebenen Art so zu verbessern, daß bei einer Erweiterung des Systems das Steuergerät auf einfache Weise entsprechend angepaßt werden kann.

Diese Aufgabe wird bei einem chirurgischen Steuergerät der eingangs beschriebenen Art erfindungsgemäß dadurch gelöst, daß das Steuergerät ein Taschencomputer ist.

Taschencomputer sind klein und handlich und können mit entsprechenden Programmen zur Bedienung eines chirurgischen Systems beziehungsweise chirurgischer Geräte ausgestattet werden. Derartige Taschencomputer, auch häufig als PDA (Personal Digital Assistant) bezeichnet, können auf einfache Weise an ein erweitertes chirurgisches System angepaßt werden, und zwar durch Erweiterung der entsprechenden Steuer- und/oder Regelungssoftware. Zudem sind keine Bedienelementerweiterungen mechanischer Art erforderlich, da handelsübliche Taschencomputer mit einer betätigbaren Graphikanzeige ausgestattet sind, einem sogenannten Touch Screen. Auf der Anzeige lassen sich beliebige und auch neue Schaltelemente definieren und darstellen, die mittels eines Eingabewerkzeugs, beispielsweise einem Zeigestab oder einem Finger, betätigbar sind. Des weiteren hat ein als Steuergerät verwendeter Taschencomputer den Vorteil, daß die Bedienung des Systems auch mit unterschiedlichen Arten von Taschencomputern vorgenommen werden kann, das heißt, eine Bedienperson kann unter Umständen den von ihr bevorzugten Taschencomputer auswählen und durch Aufspielen der benötigen Steuerund/oder Regelungssoftware den Taschencomputer zum Steuergerät des Systems zu machen. Im Sinne dieser Anmeldung sind unter Taschencomputern auch sogenannte Pocket PCs, Tablet PCs sowie Organizer zu verstehen, die alle im wesentlichen die oben beschriebenen Vorteile aufweisen.

Günstig ist es, wenn der Taschencomputer ein Handheld Personal Computer ist. Als Beispiele für derartige Handheld Personal Computer seien Palm® , Sony® Clie® , IBM® (workpad® ) und Casio® Cassiopeia® genannt. Auch alle weiteren, derartigen Geräte sind erfindungsgemäß als Steuergeräte tauglich.

Denkbar wäre es, daß eine drahtlose Kommunikation zwischen dem Steuergerät und dem chirurgischen System über Infrarot oder Ultraschallstrahlung stattfindet. Besonders vorteilhaft ist es jedoch, wenn der Taschencomputer eine Funk-Schnittstelle aufweist. Mit der Funk-Schnittstelle wird eine Übertragung von Steuer- und/oder Regelungssignalen vom Steuergerät auf das System und zum Empfang derselben ermöglicht. Ferner läßt sich so auch auf bewährte Funkübertragungsstandards zurückgreifen, was einen einfachen Austausch der Steuergeräte ermöglicht, und zwar durch Austausch von Taschencomputern unterschiedlicher Hersteller.

Um individuelle Lösungen für eine Kombination von Steuergerät und System bereitstellen zu können, ist es günstig, wenn die Funk-Schnittstelle mit dem Taschencomputer lösbar verbindbar ist. Es können so je nach Bedarf entsprechende Funk-Schnittstellen mit dem Taschencomputer verbunden werden, die eine Kommunikation mit den Geräten des Systems ermöglichen.

Es kann jedoch auch vorteilhaft sein, wenn die Funk-Schnittstelle in den Taschencomputer integriert ist. Auf diese Weise kann das Steuergerät am kompaktesten ausgebildet werden.

Gemäß einer bevorzugten Ausführungsform der Erfindung kann vorgesehen sein, daß die Funk-Schnittstelle eine Bluetooth-Schnittstelle ist. Die Verwendung von Bluetooth hat insbesondere den Vorteil, daß durch diese Technologie jedes Gerät individuell erkannt werden kann und somit eine hohe Systemsicherheit besteht. Es kann auf diese Weise vermieden werden, daß andere Geräte angesteuert werden können, die nicht für das System zugelassen sind. Im übrigen gestattet es die Bluetooth-Technologie, eine sichere Funkübertragung in Räumen zu gewährleisten, beispielsweise in einem Operationssaal.

Vorzugsweise ist der Taschencomputer derart programmiert, daß das mindestens eine chirurgische Gerät von einer Bedienperson individuell aufruf-, steuerund/oder regelbar ist. Mit dem Taschencomputer kann so auf das System als Ganzes und/oder auch auf einzelne Geräte direkt zugegriffen werden. Dabei ist es denkbar, daß mit dem Steuergerät ein Gerät eingestellt und konfiguriert wird, das wiederum weitere, für einen bestimmten Einsatzzweck benötigte Geräte zu- oder abschaltet und entsprechend einschaltet und/oder konfiguriert, wodurch dann auch das System als Ganzes einstell- und/oder konfigurierbar ist.

Günstig ist es, wenn das Steuergerät mit einer chirurgischen Haltevorrichtung lösbar verbindbar ist. Das Steuergerät kann so an einem definierten Ort, beispielsweise in einem Operationssaal, angeordnet werden. Ferner kann es, je nach Bedarf auch abgenommen werden, beispielsweise wenn ein Patient mit einem Gerät verbunden bleiben und in einen anderen Raum verlegt werden muß.

Damit das Steuergerät auch in sterilen Umgebungen eingesetzt werden kann, ist es günstig, wenn ein steriler oder sterilisierbarer Überzug für das Steuergerät vorgesehen ist.

Die eingangs gestellte Aufgabe wird ferner bei einem chirurgischen System der eingangs beschriebenen Art erfindungsgemäß dadurch gelöst, daß das Steuergerät eines der oben beschriebenen Steuergeräte ist. Die oben beschriebenen Steuergeräte lassen sich auf einfache Weise an unterschiedliche chirurgische Systeme adaptieren, insbesondere durch Austausch der Steuer- und/oder Regelungssoftware des Steuergeräts für das System.

Damit das mindestens eine chirurgische Gerät mit dem Steuergerät kommunizieren kann, weist es vorzugsweise eine Funk-Schnittstelle auf. Dabei ist es vorteilhaft, wenn das mindestens eine chirurgische Geräte eine Bluetooth-Schnittstelle aufweist. Durch die Bluetooth-Technologie kann insbesondere vermieden werden, daß mit dem Steuergerät Geräte nicht zugelassener Hersteller in das chirurgische System integriert werden können.

Vorteilhafterweise ist die Bluetooth-Schnittstelle in das chirurgische Gerät integriert. Es läßt sich so ein Platzbedarf für die Funkübertragung minimieren. Zudem werden zusätzliche Kontakte vermieden, die verunreinigt werden und dadurch eine Funktion der miteinander verbundenen Elemente nicht immer gewährleisten zu können.

Um defekte Schnittstellen schnell und einfach austauschen oder auch um verbesserte oder andere Schnittstellen vorsehen zu können, ist es günstig, wenn die Bluetooth-Schnittstelle mit dem mindestens einen chirurgischen Gerät lösbar verbindbar ist.

Chirurgische Geräte weisen häufig eine serielle Schnittstelle auf, beispielsweise eine RS232-Schnittstelle. Damit diese Geräte auch in Verbindung mit einem erfindungsgemäßen chirurgischen System verwendet werden können, ist es vorteilhaft, wenn die Bluetooth-Schnittstelle mit einer seriellen Schnittstelle des mindestens einen chirurgischen Geräts verbindbar ist. Dadurch können herkömmliche Geräte auf einfache Weise auf- beziehungsweise nachgerüstet werden, um mit dem erfindungsgemäßen chirurgischen System zum Einsatz zu kommen.

Besonders günstig ist es, wenn die serielle Schnittstelle eine RS232-Schnittstelle ist. Derartige Schnittstellen sind bei sehr vielen chirurgischen Geräten standardmäßig vorhanden, um eine Vernetzung des Geräts mit weiteren Geräten zu einem chirurgischen System zu ermöglichen.

Vorteilhafterweise umfaßt das chirurgische System mehrere Geräte. Diese können direkt nebeneinander stehen oder räumlich voneinander getrennt sein.

Gemäß einer bevorzugten Ausführungsform der Erfindung kann vorgesehen sein, daß die mehreren Geräte zur drahtlosen Kommunikation untereinander und mit dem Steuergerät ausgebildet sind. Auf diese Weise ist es möglich, auch Befehle des Steuergeräts zu einem der Geräte zu übertragen, wobei dann das vom Steuergerät angesteuerte Gerät mit einem oder mehreren weiteren Geräten kommunizieren und diese einstellen, konfigurieren oder entsprechend betreiben kann.

Günstigerweise sind die mehreren Geräten derart zur drahtlosen Kommunikation ausgebildet, daß ein Steuer- und/oder Regelungsbefehl des Steuergeräts von einem der mehreren Geräte an ein anderes der mehreren Geräte drahtlos und/oder kabelgebunden weiterleitbar ist. Auf diese Weise ist es insbesondere auch möglich, daß nur zwischen dem Steuergerät und einem der mehreren Geräte eine drahtlose Kommunikation vorgesehen ist, die anderen Geräte jedoch von dem drahtlos durch das Steuergerät angesteuerten Gerät entsprechend angesteuert werden, und zwar drahtlos und/oder kabelgebunden.

Vorteilhafterweise ist das Steuergerät mit dem Steuergerät entsprechend seinem Einsatzzweck konfigurierbar und sind die mehreren Geräte entsprechend aktivierbar und/oder einstellbar. Dadurch kann das System in gewünschter Weise verwendet werden.

Vorzugsweise sind die mehreren Geräte räumlich unabhängig voneinander angeordnet. Dies erlaubt es, einzelne Geräte des Systems beliebig auszutauschen, beispielsweise auch Geräte unterschiedlicher Hersteller in das System zu integrieren.

Günstigerweise sind alle oder ein Teil der chirurgischen Geräte in einem gemeinsamen Gehäuse angeordnet. Derartige Geräte, die in einem gemeinsamen Gehäuse angeordnet sind, werden häufig auch als Baugruppen bezeichnet. Sie können vom Steuergerät beispielsweise direkt und unabhängig voneinander oder auch gemeinsam angesteuert werden. Die Anordnung in einem gemeinsamen Gehäuse hat zudem den Vorteil, daß in der Regel nur eine einzige Strom- oder sonstige Energieversorgung für mehrere Baugruppen erforderlich ist.

Gemäß einer bevorzugten Ausführungsform der Erfindung kann vorgesehen sein, daß das System mindestens eines der nachfolgend genannten Geräte umfaßt: eine Kamera, eine Dokumentationseinheit zum Speichern von Geräte und/oder Systemdaten, ein Hochfrequenz-Gerät, ein Insufflator, eine Pumpe, ein Motorsteuergerät, ein Schnittstellengerät.

Damit das System auch auf einfache Weise mit einem Computernetzwerk oder beliebigen Arten von Datennetzen verbunden werden kann, ist es vorteilhaft, wenn das Schnittstellengerät einen Ethernet- und/oder SIOS-Anschluß aufweist. Über das Schnittstellengerät können Daten des Systems und/oder des Steuergeräts zu dem jeweiligen, an das Schnittstellengerät angeschlossenen Computernetzwerk oder Datennetz übertragen werden.

Die nachfolgende Beschreibung einer bevorzugten Ausführungsform der Erfindung dient im Zusammenhang mit der Zeichnung der näheren Erläuterung. Es zeigen:
- Figur 1:: eine schematische Darstellung eines chirurgischen Systems; und
- Figur 2:: ein Ausführungsbeispiel eines chirurgischen Steuergeräts.

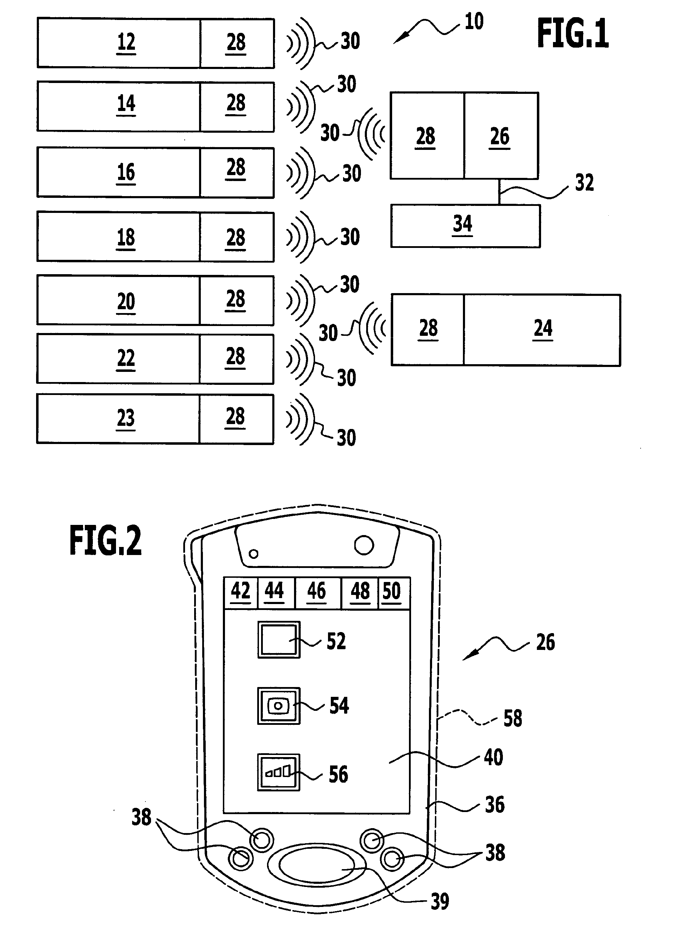

In Figur 1 ist schematisch ein insgesamt mit dem Bezugszeichen 10 versehenes chirurgisches System dargestellt. Es umfaßt eine Mehrzahl chirurgischer Geräte 12, 14, 16, 18, 20, 22 und 23, ein Schnittstellen-Gerät 24 sowie ein Steuergerät 26. Alle genannten Geräte 12 bis 26 weisen jeweils eine integrierte Bluetooth-Schnittstelle 28 auf, mit der elektromagnetische Strahlung definierter Wellenlänge beziehungsweise in einem vorgegebenen Frequenzband abgestrahlt und empfangen werden kann. Das Steuergerät kann zudem mit einem in Figur 1 nicht dargestellten sterilen Überzug versehen sein. Es ist ferner mittels einer schematisch eingezeichneten Verbindungsvorrichtung 32 mit einer OP-Tisch-Halterung lösbar verbindbar.

Eine Kommunikation zwischen dem Steuergerät 26 sowie den chirurgischen Geräten 12 bis 23 und dem Schnittstellengerät 24 erfolgt drahtlos. Mit dem Steuergerät kann jedes der Geräte 12 bis 24 individuell angesteuert, eingestellt und/oder konfiguriert werden. Zudem kann mit dem Steuergerät auch ein einzelnes Gerät angesteuert und konfiguriert werden, welches dann wiederum drahtlos weitere Geräte hinzuschaltet, abschaltet, einstellt und/oder konfiguriert.

Bei den schematisch in Figur 1 dargestellten Geräten 12 bis 23 handelt es sich beispielsweise um eine Kamera, eine Dokumentationseinheit zum Speichern von Geräte- und/oder Systemdaten, eine Lichtquelle, ein Hochfrequenz-Gerät, ein Insufflator, eine Pumpe, ein Motorsteuergerät beziehungsweise ein chirurgisches Motorensystem und ein Schnittstellen-Gerät. Die Liste der beispielhaft genannten Geräte ist nicht abschließend. Grundsätzlich kann jedes beliebige weitere Gerät, sei es chirurgisch oder auch nicht, in das chirurgische System integriert werden.

Beim Steuergerät 26 handelt es sich um einen Taschencomputer, beispielsweise einen wie in Figur 2 dargestellten PDA (Personal Digital Assistant). Das Steuergerät 26 weist ein Gehäuse 36, mehrere mechanische Bedienelemente 38 und 39 sowie eine Anzeigevorrichtung in Form eines Touch Screen-Displays 40 auf. Ferner enthält es einen nicht dargestellten Datenspeicher, der insbesondere über einsteckbare Speicherkarten erweiterbar ist. Über die nicht dargestellten Speicherkarten können insbesondere Daten transferiert sowie Programme in das Steuergerät 26 geladen werden. Softwaremäßig sind auf dem Touch Screen-Display 40 beliebige Schaltfelder 42, 44, 46, 48 und 50 darstellbar, die insbesondere einzelnen Geräten oder einer Kombination mehrerer Geräte zugeordnet sein können. Beispielsweise läßt sich über das Schaltfeld 46 ein Hochfrequenz-Gerät aufrufen und mit dann mit entsprechend erscheinenden Konfigurationsschaltflächen 52, 54 und 56 einstellen.

Beispielsweise kann für einen Videobetrieb mit dem Schaltfeld 42 eine Kamera eingestellt werden. Über die Konfigurationsschaltfläche 52 kann zum Beispiel ein Weiß-Abgleich der Kamera, über die Konfigurationsschaltfläche 54 ein Betriebsmodus der Kamera und über die Konfigurationsschaltfläche 56 eine Verstärkung der Kamera eingestellt werden.

Das Gehäuse 36 des Steuergeräts 26 ist durch einen sterilisierbaren oder sterilen Überzug 58 zum Einsatz in einem sterilen Operationsbereich geeignet.

Als Steuergerät 26 lassen sich grundsätzlich alle handelsüblichen Taschencomputer verwenden, welche eine integrierte Bluetooth-Schnittstelle 28 aufweisen oder zum Verbinden mit einer ansteckbaren Bluetooth-Schnittstelle 28 geeignet sind.

Ferner sind als Steuergerät 26 alle Arten von Taschencomputern denkbar, insbesondere Handheld-Personalcomputer, Pocket PCs, Tablet PCs sowie Organizer, mit denen eine drahtlose Datenübertragung zum Steuern und/oder Regeln des chirurgischen Systems 10 möglich ist.

## Patentansprüche

1. Chirurgisches Steuergerät (26) für ein mindestens ein chirurgisches Gerät (12, 14, 16, 18, 20, 22, 23, 24) umfassendes chirurgisches System (10), wobei das chirurgische System (10) und/oder das mindestens eine chirurgische Gerät (12, 14, 16, 18, 20, 22, 23, 24) mit dem Steuergerät (26) drahtlos steuer- und/oder regelbar ist bzw. sind, **dadurch gekennzeichnet, daß** das Steuergerät (26) ein Taschencomputer ist.

2. Steuergerät nach Anspruch 1, **dadurch gekennzeichnet, daß** der Taschencomputer (26) ein Handheld Personalcomputer ist.

3. Steuergerät nach einem der voranstehenden Ansprüche, **dadurch gekennzeichnet, daß** der Taschencomputer (26) eine Funk-Schnittstelle (28) aufweist.

4. Steuergerät nach Anspruch 3, **dadurch gekennzeichnet, daß** die Funk-Schnittstelle (28) mit dem Taschencomputer (26) lösbar verbindbar ist.

5. Steuergerät nach Anspruch 3, **dadurch gekennzeichnet, daß** die Funk-Schnittstelle (28) in den Taschencomputer (26) integriert ist.

6. Steuergerät nach einem der Ansprüche 3 bis 5, **dadurch gekennzeichnet, daß** die Funk-Schnittstelle (28) eine Bluetooth-Schnittstelle ist.

7. Steuergerät nach einem der voranstehenden Ansprüche, **dadurch gekennzeichnet, daß** der Taschencomputer (26) derart programmiert ist, daß das mindestens eine chirurgische Gerät (12, 14, 16, 18, 20, 22, 23, 24) von einer Bedienperson individuell aufruf-, steuer- und/oder regelbar ist.

8. Steuergerät nach einem der voranstehenden Ansprüche, **dadurch gekennzeichnet, daß** das Steuergerät (26) mit einer chirurgischen Haltevorrichtung (34) lösbar verbindbar ist.

9. Steuergerät nach einem der voranstehenden Ansprüche, **dadurch gekennzeichnet, daß** ein steriler oder sterilisierbarer Überzug (58) für das Steuergerät (26) vorgesehen ist.

10. Chirurgisches System (10) umfassend mindestens ein chirurgisches Gerät (12, 14, 16, 18, 20, 22, 23, 24) und ein chirurgisches Steuergerät (26) zum drahtlosen Steuern und/oder Regeln des chirurgischen Systems (10), **dadurch gekennzeichnet, daß** das Steuergerät (26) ein Steuergerät (26) nach einem der voranstehenden Ansprüche ist.

11. System nach Anspruch 10, **dadurch gekennzeichnet, daß** das mindestens eine chirurgische Gerät (12, 14, 16, 18, 20, 22, 23, 24) eine Bluetooth-Schnittstelle (28) aufweist.

12. System nach Anspruch 11, **dadurch gekennzeichnet, daß** die Bluetooth-Schnittstelle (28) in das mindestens eine chirurgische Gerät (12, 14, 16, 18, 20, 22, 23, 24) integriert ist.

13. System nach Anspruch 11, **dadurch gekennzeichnet, daß** die Bluetooth-Schnittstelle (28) mit dem mindestens einen chirurgischen Gerät (12, 14, 16, 18, 20, 22, 23, 24) lösbar verbindbar ist.

14. System nach einem der Ansprüche 11 bis 13, **dadurch gekennzeichnet, daß** die Bluetooth-Schnittstelle (28) mit einer seriellen Schnittstelle des mindestens einen chirurgischen Geräts (12, 14, 16, 18, 20, 22, 23, 24) verbindbar ist.

15. System nach Anspruch 14, **dadurch gekennzeichnet, daß** die serielle Schnittstelle eine RS232-Schnittstelle ist.

16. System nach einem der Ansprüche 10 bis 15, **dadurch gekennzeichnet, daß** das chirurgische System (10) mehrere chirurgische Geräte (12, 14, 16, 18, 20, 22, 23, 24) umfaßt.

17. System nach Anspruch 16, **dadurch gekennzeichnet, daß** die mehreren chirurgischen Geräte (12, 14, 16, 18, 20, 22, 23, 24) zur drahtlosen Kommunikation untereinander und mit dem Steuergerät (26) ausgebildet sind.

18. System nach einem der Ansprüche 16 oder 17, **dadurch gekennzeichnet, daß** die mehreren chirurgischen Geräte (12, 14, 16, 18, 20, 22, 23, 24) derart zur drahtlosen Kommunikation ausgebildet sind, daß ein Steuerund/oder Regelbefehl des Steuergeräts (26) von einem der mehreren chirurgischen Geräte (12, 14, 16, 18, 20, 22, 23, 24) an ein anderes der mehreren chirurgischen Geräte (12, 14, 16, 18, 20, 22, 23, 24) drahtlos und/oder kabelgebunden weiterleitbar ist.

19. System nach einem der Ansprüche 16 bis 18, **dadurch gekennzeichnet, daß** das System (10) mit dem Steuergerät (26) entsprechend seinem Einsatzzweck konfigurierbar ist und daß die mehreren chirurgischen Geräte (12, 14, 16, 18, 20, 22, 23, 24) entsprechend aktivierbar und/oder einstellbar sind.

20. System nach einem der Ansprüche 16 bis 19, **dadurch gekennzeichnet, daß** die mehreren chirurgischen Geräte (12, 14, 16, 18, 20, 22, 23, 24) räumlich unabhängig voneinander angeordnet sind.

21. System nach einem der Ansprüche 16 bis 20, **dadurch gekennzeichnet, daß** alle oder ein Teil der chirurgischen Geräte (12, 14, 16, 18, 20, 22, 23, 24) in einem gemeinsamen Gehäuse angeordnet sind.

22. System nach einem der Ansprüche 10 bis 21, **dadurch gekennzeichnet, daß** das System (10) mindestens eines der nachfolgend genannten chirurgischen Geräte (12, 14, 16, 18, 20, 22, 23, 24) umfaßt: eine Kamera (12), eine Dokumentationseinheit (14) zum Speichern von Geräteund/oder Systemdaten, eine Lichtquelle (16), ein Hochfrequenz-Gerät (18), ein Insufflator (20), eine Pumpe (22), ein Motorsteuergerät (23) ein Schnittstellen-Gerät (24).

23. System nach Anspruch 22, **dadurch gekennzeichnet, daß** das Schnittstellengerät (24) einen Ethernet- und/oder SIOS-Anschluß aufweist.
